# EUROPÄISCHE PATENTSCHRIFT

(11) **EP 1 442 000 B1**
(45) Veröffentlichungstag und Bekanntmachung des Hinweises auf die Patenterteilung: **25.03.2009**
(21) Anmeldenummer: 02801334.0
(22) Anmeldetag: 14.10.2002
(51) Int. Cl.: C07C 5/48, C07C 5/327

(54) **VERFAHREN ZUR DEHYDRIERUNG VON C 2-C 30-ALKANEN**
METHOD FOR DEHYDROGENATING C 2-C 30-ALKANES
PROCEDE POUR DESHYDROGENER DES ALCANES C 2-C 30

(30) Priorität: 15.10.2001 DE 10150811
(43) Veröffentlichungstag der Anmeldung: 04.08.2004
(73) Patentinhaber: BASF SE, 67056 Ludwigshafen (DE)
(72) Erfinder: WALSDORFF, Christian, 67059 Ludwigshafen (DE); SCHINDLER, Götz-Peter, 68219 Mannheim (DE); MACHHAMMER, Otto, 68163 Mannheim (DE); HARTH, Klaus, 67317 Altleiningen (DE)
(74) Vertreter: Isenbruck, Günter
(86) Internationale Anmeldenummer: PCT/EP2002/011467
(87) Internationale Veröffentlichungsnummer: WO 2003/033440

(56) Entgegenhaltungen:
- WO-A-01/56960
- US-A- 2 376 532
- US-A- 4 788 371
- US-A- 5 733 518

## Beschreibung

Die Erfindung betrifft ein Verfahren zur heterogen katalysierten Dehydrierung von dehydrierbaren C₂-C₃₀-Alkanen.

Dehydrierte Kohlenwasserstoffe werden als Ausgangsstoffe für zahlreiche industrielle Verfahren in großen Mengen benötigt. Beispielsweise finden dehydrierte Kohlenwasserstoffe bei der Herstellung von Detergentien, klopffestem Benzin und pharmazeutischen Produkten Verwendung. Ebenso werden zahlreiche Kunststoffe durch Polymerisation von Olefmen hergestellt.

Beispielsweise werden aus Propen Acrylnitril, Acrylsäure oder C₄-Oxoalkohole hergestellt. Propen wird derzeit überwiegend durch Steamcracken oder durch katalytisches Cracken geeigneter Kohlenwasserstoffe oder Kohlenwasserstoffgemische wie Naphtha hergestellt.

Propen kann darüber hinaus durch heterogen katalysierte Dehydrierung von Propan hergestellt werden.

Um bei heterogen katalysierten Dehydrierungen akzeptable Umsätze auch schon bei einmaligem Reaktor-Durchgang zu erreichen, muss in der Regel bei relativ hohen Reaktionstemperaturen gearbeitet werden. Typische Reaktionstemperaturen für Gasphasendehydrierungen liegen bei 300 bis 700°C. Pro Molekül Kohlenwasserstoff wird dabei in der Regel ein Molekül Wasserstoff erzeugt.

Die Dehydrierung von Kohlenwasserstoffen verläuft endotherm. Die für die Einstellung eines gewünschten Umsatzes benötigte Dehydrierwärme muss entweder dem Reaktionsgas vorab und/oder im Verlauf der katalytischen Dehydrierung zugeführt werden. Bei den meisten bekannten Dehydrierverfahren wird die Dehydrierwärme außerhalb des Reaktors erzeugt und dem Reaktionsgas von außen zugeführt. Dies erfordert jedoch aufwendige Reaktor- und Verfahrenskonzepte und führt insbesondere bei hohen Umsätzen zu steilen Temperaturgradienten im Reaktor, mit der Gefahr einer verstärkten Nebenproduktbildung. So können beispielsweise mehrere adiabate Katalysatorbetten in nacheinander geschalteten Ringspaltreaktoren angeordnet werden. Das Reaktionsgasgemisch wird auf seinem Weg von einem Katalysatorbett zum nächsten Katalysatorbett durch Wärmeaustauscher überhitzt und kühlt sich beim nachfolgenden Reaktordurchgang wieder ab. Um mit einem solchen Reaktorkonzept zu hohen Umsätzen zu gelangen muss entweder die Anzahl der hintereinander geschalteten Reaktoren erhöht werden, oder es muss die Reaktoreingangstemperatur des Gasgemischs erhöht werden. Die dadurch bedingte Überhitzung führt zwangsläufig zu einer verstärkten Nebenproduktbildung durch Crackreaktionen. Ferner ist bekannt, das Katalysatorbett in einem Rohrreaktor anzuordnen und die Dehydrierwärme durch das Verfeuern von brennbaren Gasen außerhalb des Rohrreaktors zu erzeugen und über die Rohrwand in das Innere des Reaktors einzukoppeln. Bei diesen Reaktoren führen hohe Umsätze zu steilen Temperaturgradienten zwischen der Wand und dem Inneren des Reaktionsrohres.

Eine Alternative ist die Erzeugung der Dehydrierwärme direkt in dem Reaktionsgasgemisch der Dehydrierung durch Oxidation von bei der Dehydrierung gebildetem oder zusätzlich zugeführtem Wasserstoff oder von im Reaktionsgasgemisch enthaltenen Kohlenwasserstoffen mit Sauerstoff. Dazu wird dem Reaktionsgasgemisch entweder bereits vor dem ersten Katalysatorbett oder vor nachfolgenden Katalysatorbetten ein sauerstoffhaltiges Gas und gegebenenfalls Wasserstoff zugesetzt. Gleichzeitig wird durch den Verzicht auf die indirekte Reaktorheizung ein sehr einfaches Verfahrenskonzept verwirklicht.

US 4,758,371 beschreibt ein Verfahren zur Wasserdampfdehydrierung von dehydrierbaren Kohlenwasserstoffen in der Gasphase in Verbindung mit einem oxidativen Wiedererhitzen der Zwischenprodukte, wobei der gleiche Katalysator für die selektive Oxidation von Wasserstoff und die Wasserdampfdehydrierung eingesetzt wird. Dabei kann Wasserstoff als Co-Feed zugeführt werden. Der verwendete Katalysator enthält ein Edelmetall der Gruppe VIII, ein Alkalimetall und ein weiteres Metall aus der Gruppe B, Ga, In, Ge, Sn, und Pb auf einem anorganischen Oxidträger wie Aluminiumoxid. Das Verfahren kann ein- oder mehrstufig in einem Fest- oder Wanderbett durchgeführt werden.

US 5,733,518 beschreibt ein Verfahren zur selektiven Oxidation von Wasserstoff mit Sauerstoff in Gegenwart von Kohlenwasserstoffen wie n-Butan an einem Katalysator enthaltend ein Phosphat von Germanium, Zinn, Blei, Arsen, Antimon oder Bismut, vorzugsweise Zinn. Durch die Verbrennung des Wasserstoffs wird die für die endotherme Dehydrierung notwendige Reaktionswärme in wenigstens einer Reaktionszone erzeugt.

EP-A 0 838 534 beschreibt einen Katalysator für die wasserdampffreie Dehydrierung von Alkanen, insbesondere von Isobutan, in Gegenwart von Sauerstoff. Der verwendete Katalysator umfasst ein Platingruppenmetall, welches auf einem Träger aus Zinnoxid/Zirkoniumoxid mit mindestens 10% Zinn aufgebracht ist. Der Sauerstoffgehalt im Einsatzstrom der Dehydrierung wird so abgestimmt, dass die durch Verbrennung von Wasserstoff mit Sauerstoff erzeugte Wärmemenge gleich der für die Dehydrierung benötigten Wärmemenge ist.

WO 96/33151 beschreibt ein Verfahren zur Dehydrierung eines C₂-C₅-Alkans in Abwesenheit von Sauerstoff an einem Dehydrierkatalysator, enthaltend Cr, Mo, Ga, Zn oder ein Gruppe VIII-Metall unter gleichzeitiger Oxidation von gebildetem Wasserstoff an einem reduzierbaren Metalloxid wie den Oxiden von Bi, In, Sb, Zn, Tl, Pb oder Te. Die Dehydrierung muss dabei regelmäßig unterbrochen werden, um das reduzierte Oxid wieder mit einer Sauerstoffquelle zu reoxidieren. In US 5,430,209 ist ein entsprechendes Verfahren beschrieben, bei dem der Dehydrierungsschritt und der Oxidationsschritt nacheinander ablaufen und die dazugehörigen Katalysatoren räumlich voneinander getrennt sind. Als Katalysatoren für die selektive Wasserstoff-Oxidation kommen Oxide des Bi, Sb und Te sowie deren Mischoxide zum Einsatz.

WO 96/33150 beschreibt schließlich ein Verfahren, in dem in einer ersten Stufe ein C₂-C₅-Alkan an einem Dehydrierungskatalysator dehydriert wird, das Austrittsgas der Dehydrierungsstufe mit Sauerstoff gemischt wird und in einer zweiten Stufe über einen Oxidationskatalysator, bevorzugt Bi₂O₃, geleitet wird, wobei der gebildete Wasserstoff selektiv zu Wasser oxidiert wird, und in einer dritten Stufe das Ausgangsgas der zweiten Stufe erneut über einen Dehydrierungskatalysator geleitet wird.

Bei der Dehydrierung von Ethylbenzol zu Styrol fällt als Nebenprodukt Wasserstoff an, weltweit ca. 350 000 t pro Jahr. Dieser Wasserstoff enthält Nebenbestandteile, insbesondere Kohlenmonoxid, Kohlendioxid, Methan, Ethan und Ethylen, die eine Weiterverwendung dieses Wasserstoffs für Hydrierungen häufig nicht zulassen, da die Nebenprodukte den Hydrierungskatalysator vergiften können. Eine Reinigung des Wasserstoffstroms von den Nebenbestandteilen ist im allgemeinen jedoch nicht wirtschaftlich. Aus diesen Gründen wird der bei der Ethylbenzol-Dehydrierung anfallende Wasserstoff in der Regel verbrannt, beispielsweise zur Dampferzeugung oder Dampfüberhitzung.

Aufgabe der Erfindung ist, ein vorteilhaftes Verfahren zur Dehydrierung von Alkanen bereitzustellen. Aufgabe der Erfindung ist ferner, den bei der Ethylbenzol-Dehydrierung gebildeten Wasserstoff einer höherwertigen Verwendung zuzuführen.

Gelöst wird die Aufgabe durch ein Verfahren zur heterogen katalysierten Dehydrierung von C₂-C₃₀-Alkanen, bei dem
(i) in einem ersten Teilverfahren Ethylbenzol zu Styrol dehydriert wird, wobei ein wasserstoffhaltiger Abgasstrom gewonnen wird, und
(ii) in einem zweiten Teilverfahren ein oder mehrere C₂-C₃₀-Alkane in einer oder mehreren Reaktionszonen heterogen katalysiert zu den entsprechenden Olefinen dehydriert werden, wobei dem Reaktionsgasgemisch der Dehydrierung in
   mindestens einer Reaktionszone ein Wasserstoff enthaltender Gasstrom zugemischt wird, und dem Reaktionsgasgemisch der Alkan-Dehydrierung in mindestens einer Reaktionszone zusätzlich Sauerstoff zugemischt und der in dem Reaktionsgasgemisch enthaltene zugemischte Wasserstoff verbrannt wird, wodurch zumindest ein Teil der benötigten Dehydrierwärme in der mindestens einen Reaktionszone direkt in dem Reaktionsgasgemisch erzeugt wird,
wobei zumindest ein Teil des in der Ethylbenzol-Dehydrierung gewonnenen Wasserstoff enthaltenden Abgasstroms dem Reaktionsgasgemisch der Alkan-Dehydrierung zugemischt wird.

In dem ersten Teilverfahren wird Ethylbenzol zu Styrol dehydriert. Die Ethylbenzol-Dehydrierung erfolgt im allgemeinen in der Gasphase bei Temperaturen von 520 bis 650°C in Gegenwart eines Katalysators. Es kann bei Normaldruck, leicht erhöhtem Druck, beispielsweise bis ca. 2 bar oder bevorzugt bei verringertem Druck von ca. 0,2 bis 0,7 bar gearbeitet werden. Durch das Arbeiten bei verringertem Druck wird das Reaktionsgleichgewicht auf die Produktseite verschoben. Die Ethylbenzol-Dehydrierung wird üblicherweise in Gegenwart von Wasserdampf durchgeführt, wobei das Gewichtsverhältnis Wasserdampf : Ethylbenzol von 0,5 bis 2,5, vorzugsweise 1,0 bis 1,5 beträgt. Der zugesetzte Wasserdampf dient als Wärmeträger, stabilisiert eine mittlere Oxidationsstufe der typischerweise eisenoxidhaltigen Katalysatoren und unterstützt die Vergasung von organischen Ablagerungen auf den Katalysatoren, wodurch der Verkokung der Katalysatoren entgegengewirkt wird. Dabei werden die organischen Ablagerungen in Kohlenmonoxid und Kohlendioxid umgewandelt. Nach Verlassen des Dehydrierreaktors werden Wasser und die höhersiedenden organische Komponenten wie Styrol, unumgesetztes Ethylbenzol und als Nebenprodukte gebildetes Benzol und Toluol aus dem Reaktionsgasgemisch auskondensiert. Es verbleibt ein wasserstoffhaltiger Abgasstrom, der neben Kohlenmonoxid und Kohlendioxid noch Kohlenwasserstoffe wie Methan, Ethan und Ethylen sowie weitere Bestandteile enthalten kann.

Im allgemeinen enthält der Abgasstrom der Ethylbenzol-Dehydrierung 0,1 bis 4 Vol.-% Kohlenwasserstoffe und 1,5 bis 8 Vol.-% Kohlenstoffoxide. Typischerweise enthält der Abgasstrom der Ethylbenzol-Dehydrierung 90 bis 97 Vol.-% Wasserstoff, 0,05 bis 0,4 Vol.-% Kohlenmonoxid, 3 bis 7 Vol.-% Kohlendioxid, 0,1 bis 2 Vol.-% Methan, 0,02 bis 0,2 Vol.-% Ethan, 0,1 bis 1,5 Vol.-% Ethylen und insgesamt 0 bis 2 Vol.-% Wasserdampf,

Propan, Benzol und/oder Stickstoff, wobei die Summe aus den genannten Komponenten 100 Vol.-% ergibt.

Der wasserstoffhaltige Abgasstrom aus der Ethylbenzol-Dehydrierung wird in der Alkan-Dehydrierung eingesetzt. Es kann der gesamte Abgasstrom oder nur ein Teil davon eingesetzt werden. Darüber hinaus kann zusätzlich Wasserstoff aus anderen Quellen mitverwendet werden.

Die Gegenwart von zusätzlichem Wasserstoff im Reaktionsgasgemisch der Alkan-Dehydrierung wirkt der Verkokung des Dehydrierkatalysators entgegen, wodurch sich die Standzeit des Katalysators erhöht.

Ein Teil der Dehydrierwärme wird in mindestens einer Reaktionszone durch exotherme Reaktion von Wasserstoff in Gegenwart von Sauerstoff direkt in dem Reaktionsgasgemisch erzeugt. Im allgemeinen beträgt die insgesamt zugeführte Sauerstoffmenge, bezogen auf die Gesamtmenge des zu dehydrierenden Alkans, 0,001 bis 0,5 mol/mol, bevorzugt 0,005 bis 0,2 mol/mol, besonders bevorzugt 0,05 bis 0,2 mol/mol. Im allgemeinen wird die Menge des dem Reaktionsgasgemisch zugesetzten Sauerstoffs so gewählt, dass durch die Verbrennung des im Reaktionsgasgemisch vorhandenen Wasserstoffs und gegebenenfalls von im Reaktionsgasgemisch vorliegenden Kohlenwasserstoffen und/oder von in Form von Koks vorliegendem Kohlenstoff die für die Dehydrierung des Alkans zum Olefin benötigte Wärmemenge erzeugt wird. In besonderen Ausführungsformen kann die durch Verbrennung mit Sauerstoff erzeugte Wärme auch größer oder kleiner sein als die für die Dehydrierung des Kohlenwasserstoffs benötigte Wärme. Sauerstoff kann entweder als reiner Sauerstoff oder als sauerstoffhaltiges Gas im Gemisch mit Inertgasen wie CO₂, N₂ oder den Edelgasen eingesetzt werden. Bevorzugtes sauerstoffhaltiges Gas ist Luft. Alternativ zu molekularem Sauerstoff können auch andere sauerstoffhaltige gasförmige Oxidationsmittel, beispielsweise Distickstoffoxid oder Ozon, eingesetzt werden. Die Inertgase und die resultierenden Verbrennungsgase wirken im allgemeinen zusätzlich verdünnend und fördern damit die heterogen katalysierte Dehydrierung.

Der zur Wärmeerzeugung verbrannte Wasserstoff ist der dem Reaktionsgasgemisch zusätzlich zugesetzte Wasserstoff aus der Ethylbenzol-Dehydrierung sowie der bei der Kohlenwasserstoff-Dehydrierung gebildete Wasserstoff.

Im allgemeinen wird dem Reaktionsgasgemisch soviel Wasserstoff zugesetzt, dass das Molverhältnis H₂/O₂ im Reaktionsgasgemisch ummittelbar nach der Einspeisung 0,1 bis 200, vorzugsweise 1 bis 20, besonders bevorzugt 2 bis 10 mol/mol beträgt. Dies gilt bei mehrstufigen Reaktoren für jede Zwischeneinspeisung von Wasserstoff und Sauerstoff.

Die Wasserstoffverbrennung erfolgt katalytisch. Der in dem zweiten Teilverfahren eingesetzte Dehydrierungskatalysator katalysiert im allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform der Erfindung wird kein von dem Dehydrierungskatalysator verschiedener spezieller Oxidationskatalysator eingesetzt. In einer weiteren Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung der Kohlenwasserstoffe mit Sauerstoff zu CO und CO₂ läuft dadurch nur in untergeordnetem Maße ab, was sich deutlich positiv auf die erzielten Selektivitäten für die Bildung der Olefine auswirkt. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff in Gegenwart von Kohlenwasserstoffen katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder Wasserstoff kann an einer oder mehreren Stelle des Reaktors erfolgen.

Ein bevorzugter Katalysator, der selektiv die Verbrennung von Wasserstoff katalysiert, enthält Oxide oder Phosphate, ausgewählt aus der Gruppe bestehend aus den Oxiden oder Phosphaten von Germanium, Zinn, Blei, Arsen, Antimon oder Bismut.

Ein weiterer bevorzugter Katalysator, der die Verbrennung von Wasserstoff katalysiert, enthält ein Edelmetall der VIII. und/oder I. Nebengruppe.

Durch Verdünnung des Reaktionsgasgemisches mit Inertgas, durch Erhöhung der Reaktionstemperatur und/oder durch Absenken des Reaktionsdruckes lassen sich die thermodynamisch möglichen Grenzumsätze so weit erhöhen, dass sie deutlich über den angestrebten Reaktionsumsätzen liegen. Auf diese Weise lassen sich beispielsweise bei der Propan-Dehydrierung Raum-Zeit-Ausbeuten von über 1 kg Propen / kg Katalysator * h erzielen. Die Belastung (GHSV) des Katalysators kann bei dieser auch als Hochlastfahrweise bezeichneten Fahrweise > 4000 h⁻¹ betragen.

Der Dehydrierungskatalysators kann in an sich bekannter Weise regeneriert werden. So kann, wie oben beschrieben, dem Reaktionsgasgemisch Wasserdampf zugesetzt werden. Sich abscheidender Kohlenstoff wird unter diesen Reaktionsbedingungen nach dem Prinzip der Kohlevergasung teilweise oder vollständig entfernt.

Alternativ dazu kann von Zeit zu Zeit ein Sauerstoff enthaltendes Gas bei erhöhter Temperatur über die Katalysatorschüttung geleitet werden und der abgeschiedene Kohlenstoff abgebrannt werden.

Nach längerer Betriebsdauer wird der Dehydrierungskatalysator bevorzugt dadurch regeneriert, dass bei einer Temperatur von 300 bis 600°C, häufig bei 350 bis 500°C, zunächst ein Spülgang mit Inertgas durchführt wird und anschließend in einer ersten Regenerierstufe mit Stickstoff oder Wasserdampf verdünnte Luft über das Katalysatorbett geleitet wird. Die Katalysatorbelastung beträgt dabei vorzugsweise 50 bis 10 000 h⁻¹ und der Sauerstoffgehalt ca. 0,5 bis 2 Vol.-%. In weiteren sich anschließenden Regenerierstufen wird der Sauerstoffgehalt sukzessive auf ca. 20 Vol.-% (reine Luft) erhöht. Vorzugsweise werden 2 bis 10, besonders bevorzugt 2 bis 5 Regenerierstufen durchgeführt. Im allgemeinen wird anschließend noch mit reinem Wasserstoff oder mit einem Inertgas verdünntem Wasserstoff (Wasserstoffgehalt > 1 Vol-%) unter ansonsten gleichen Bedingungen regeneriert. Bevorzugt werden alle Regenerierstufen in Gegenwart von Wasserdampf durchgeführt.

Die Alkan-Dehydrierung nach dem zweiten Teilverfahren des erfindungsgemäßen Verfahrens kann grundsätzlich in allen aus dem Stand der Technik bekannten Reaktortypen und Fahrweisen durchgeführt werden. Die zusätzliche Einspeisung von Wasserstoff und Sauerstoff führt dazu, dass zumindest ein Teil der Reaktionswärme bzw. der Energie, die zum Aufheizen des Reaktionsgasgemisches erforderlich ist, durch die direkte Verbrennung aufgebracht wird und nicht indirekt über Wärmeaustauscher übertragen werden muss.

Eine ausführliche Beschreibung von geeigneten Reaktortypen und Fahrweisen enthält auch "Catalytica® Studies Division, Oxidative Dehydrogenation and Alternative Dehydrogenation Processes, Study Number 4192 OD, 1993, 430 Ferguson Drive, Mountain View, California, 94043-5272 U.S.A."

Eine geeignete Reaktorform ist der Festbettrohr- oder Rohrbündelreaktor. Bei diesen befindet sich der Katalysator (Dehydrierungs- und gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Das indirekte Aufheizen des Reaktionsgases kann erfindungsgemäß in vorteilhafter Weise mit dem direkten Aufheizen durch Verbrennung in dem Reaktionsgasgemisch gekoppelt werden. Durch Kopplung der direkten Wärmeeinbringung mit der indirekten Wärmeinbringung ist eine annähernd isotherme Reaktionsführung erreichbar. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 700°C, vorzugsweise im Bereich von 400 bis 700°C. Der Arbeitsdruck liegt üblicherweise zwischen 0,5 und 8 bar, häufig zwischen 1 und 2 bar bei Verwendung einer geringen Wasserdampfverdünnung (entsprechend dem BASF-Linde-Verfahren), aber auch zwischen 3 und 8 bar bei Verwendung einer hohen Wasserdampfverdünnung (entsprechend dem sogenannten "steam active reforming process" (STAR-Prozess) von Phillips Petroleum Co., siehe US 4,902,849, US 4,996,387 und US 5,389,342). In der Regel verlässt das Produktgemisch das Reaktionsrohr mit einer 50 bis 100°C tieferen Temperatur. Typische Katalysatorbelastungen mit Propan liegen bei 500 bis 2000 h⁻¹. Die Katalysatorgeometrie kann beispielsweise kugelförmig oder zylindrisch (hohl oder voll) sein.

Die Alkan-Dehydrierung nach dem zweiten Teilverfahren des erfindungsgemäßen Verfahrens kann in einem Wanderbett-Reaktor durchgeführt werden. Beispielsweise kann das Katalysator-Wanderbett in einem Radialstromreaktor untergebracht sein. In diesem bewegt sich der Katalysator langsam von oben nach unten, während das Reaktionsgasgemisch radial fließt. Diese Verfahrensweise wird beispielsweise im sogenannten UOP-Oleflex-Dehydrierverfahren angewandt. Da die Reaktoren bei diesem Verfahren quasi adiabat betrieben werden, ist es zweckmäßig, mehrere Reaktoren hintereinander geschaltet zu betreiben (in typischer Weise bis zu vier Reaktoren). Vor oder in jedem Reaktor wird das Eintrittsgasgemisch durch Verbrennung in Gegenwart des zugeführten Sauerstoffs auf die erforderliche Reaktionstemperatur aufgeheizt. Durch die Verwendung mehrerer Reaktoren lassen sich große Unterschiede der Temperaturen des Reaktionsgasgemisches zwischen Reaktoreingang und Reaktorausgang vermeiden und trotzdem hohe Gesamtumsätze erzielen.

Wenn das Katalysatorbett den Wanderbettreaktor verlassen hat, wird es der Regenerierung zugeführt und anschließend wiederverwendet. Der eingesetzte Dehydrierungskatalysator weist im allgemeinen Kugelform auf. Der Arbeitsdruck liegt typischerweise bei 2 bis 5 bar. Das Molverhältnis von Wasserstoff zu Propan beträgt vorzugsweise von 0,1 bis 10. Die Reaktionstemperaturen liegen bevorzugt bei 550 bis 660°C.

Eine Alkan-Dehydrierung nach dem zweiten Teilverfahren des erfindungsgemäßen Verfahrens kann auch, wie in Chem. Eng. Sci. 1992 b, 47 (9-11) 2313 beschrieben, heterogen katalysiert im Wirbelbett durchgeführt werden, wobei der Kohlenwasserstoff nicht verdünnt wird. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Wasserstoff enthaltenden Co-Feeds und eines Sauerstoff enthaltenden Co-Feeds kann auf die Vorerhitzer verzichtet werden, und die benötigte Wärme direkt im Reaktorsystem durch Verbrennung von Wasserstoff in Gegenwart von Sauerstoff erzeugt werden.

In einer besonders bevorzugten Ausführungsform des erfindungsgemäßen Verfahrens wird die Alkan-Dehydrierung nach dem zweiten Teilverfahren in einem Hordenreaktor durchgeführt. Dieser enthält ein oder mehrere aufeinander folgende Katalysatorbetten. Die Anzahl der Katalysatorbetten kann 1 bis 20, zweckmäßigerweise 2 bis 8, insbesondere 4 bis 6 betragen. Die Katalysatorbetten werden vorzugsweise radial oder axial vom Reaktionsgas durchströmt. Im allgemeinen wird ein solcher Hordenreaktor mit einem Katalysatorfestbett betrieben.

Im einfachsten Fall sind die Katalysatorfestbetten in einem Schachtofenreaktor axial oder in den Ringspalten von zentrisch ineinander gestellten zylindrischen Gitterrosten angeordnet. Ein Schachtofenreaktor entspricht einer Horde. Die Durchführung der Dehydrierung in einem einzelnen Schachtofenreaktor ist möglich, aber weniger bevorzugt.

Bei einer Fahrweise ohne Sauerstoff als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherrippen oder durch Durchleiten durch mit heißen Brenngasen beheizte Rohre.

In der bevorzugten Variante des erfindungsgemäßen Verfahren wird eine Zwischenerhitzung des Reaktionsgasgemischs zumindest teilweise auf direktem Weg durchgerührt. Dazu wird dem Reaktionsgasgemisch entweder bereits vor Durchströmen des ersten Katalysatorbetts und/oder zwischen den nachfolgenden Katalysatorbetten in begrenztem Umfang Wasserstoff enthaltendes Abgas der Ethylbenzol-Dehydrierung und molekularer Sauerstoff zugesetzt. An dem Katalysator werden so in begrenztem Umfang zugesetzter Wasserstoff, aber auch im Verlauf der Dehydrierung gebildeter Wasserstoff, im Reaktionsgasgemisch enthaltene Kohlenwasserstoffe und/oder bereits auf der Katalysatoroberfläche abgeschiedener Koks oder kohleähnliche Verbindungen verbrannt. Die dabei freigesetzte Reaktionswärme ermöglicht so eine nahezu isotherme Betriebsweise der heterogen katalysierten Kohlenwasserstoffdehydrierung.

In einer Ausführungsform des erfindungsgemäßen Verfahrens erfolgt eine Zwischeneinspeisung von sauerstoffhaltigem Gas und von Wasserstoff enthaltendem Dehydrierabgas vor jeder Horde des Hordenreaktors. In einer bevorzugten Ausführungsform ist hinter jeder Einspeisungsstelle eine Schicht aus einem speziellen Oxidationskatalysator vorhanden, gefolgt von einer Schicht aus dem Dehydrierungskatalysator. In einer zweiten bevorzugten Ausführungsform ist kein spezieller Oxidationskatalysator vorhanden.

Die Dehydriertemperatur beträgt im allgemeinen 400 bis 800 °C, der Druck im allgemeinen 0,2 bis 5 bar, bevorzugt 0,5 bis 2 bar, besonders bevorzugt 1 bis 1,5 bar. Die Belastung (GHSV) beträgt im allgemeinen 500 bis 2000 h⁻¹, bei Hochlastfahrweise auch bis zu 16000 h⁻¹, bevorzugt 4000 bis 16000 h⁻¹.

Die in dem zweiten Teilverfahren des erfindungsgemäßen Verfahrens eingesetzten Dehydrierungskatalysatoren weisen im allgemeinen einen Träger und eine Aktivmasse auf. Der Träger besteht dabei aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Zur Herstellung der Dehydrierungskatalysatoren können Precursoren für Oxide des Zirkons, Siliciums, Aluminiums, Titans, Magnesiums, Lanthans oder Cers, die sich durch Calcinieren in die Oxide umwandeln lassen, eingesetzt werden. Diese können nach bekannten Verfahren, zum Beispiel nach dem Sol-Gel-Verfahren, Fällung der Salze, Entwässern der entsprechenden Säuren, Trockenmischen, Aufschlämmen oder Sprühtrocknen hergestellt werden. Zum Beispiel kann zur Herstellung eines ZrO₂•Al₂O₃•SiO₂-Mischoxides zunächst ein wasserreiches Zirkonoxid der allgemeinen Formel ZrO₂•xH₂O durch Fällung eines geeigneten Zirkon enthaltenden Precursors hergestellt werden. Geeignete Precursoren des Zirkons sind zum Beispiel Zr(NO₃)₄, ZrOCl₂, oder ZrCl₄ . Die Fällung selbst erfolgt durch Zugabe einer Base wie zum Beispiel NaOH, KOH, Na₂CO₃ und NH₃ und ist beispielsweise in der EP-A 0 849 224 beschrieben.

Zur Herstellung eines ZrO₂•SiO₂-Mischoxides kann der zuvor erhaltene Zirkon enthaltende Precursor mit einem Silizium enthaltenden Precursor gemischt werden. Gut geeignete Precursoren für SiO₂ sind zum Beispiel wasserhaltige Sole des SiO₂ wie Ludox^{™}. Die Mischung der beiden Komponenten kann beispielsweise durch einfaches mechanisches Vermischen oder durch Sprühtrocknen in einem Sprühturm erfolgen.

Zur Herstellung eines ZrO₂•SiO₂•Al₂O₃-Mischoxides kann die wie oben beschrieben erhaltene SiO₂•ZrO₂-Pulvermischung mit einem Aluminium enthaltenden Precursor versetzt werden. Dies kann zum Beispiel durch einfaches mechanisches Mischen in einem Kneter erfolgen. Die Herstellung ZrO₂•SiO₂•Al₂O₃-Mischoxides kann aber auch in einem einzigen Schritt durch Trockenmischung der einzelnen Precursoren erfolgen.

Die Träger für die Dehydrierungskatalysatoren haben unter anderem den Vorteil, dass sie sich leicht verformen lassen. Dazu wird die erhaltene Pulvermischung im Kneter mit einer konzentrierten Säure versetzt und dann in einen Formkörper, z.B. mittels einer Strangpresse oder eines Extruders, überführt.

Die Dehydrierungskatalysatoren besitzen in besonderen Ausgestaltungsformen eine definierte Porenstruktur. Bei der Verwendung von Mischoxiden besteht die Möglichkeit der gezielten Beeinflussung der Porenstruktur. Die Korngröße der verschiedenen Precursoren beeinflussen das Porengefüge. So lassen sich beispielsweise über die Verwendung von Al₂O₃ mit einem geringen Glühverlust und einer definierten Korngrößenzusammensetzung Makroporen im Gefüge erzeugen. Bewährt hat sich in diesem Zusammenhang die Verwendung von Al₂O₃ mit einem Glühverlust von etwa 3% (z. B. Puralox®).

Eine weitere Möglichkeit zur gezielten Herstellung der Träger mit speziellen Porenradienverteilungen für die erfindungsgemäß eingesetzten Dehydrierungskatalysatoren besteht in der Zugabe verschiedener Polymere während der Herstellung, die durch Calcinierung teilweise oder vollständig entfernt werden, wobei Poren in definierten Porenradienbereichen entstehen. Die Mischung der Polymere und der Oxid-Precursoren kann beispielsweise durch einfaches mechanisches Vermischen oder durch Sprühtrocknen in einem Sprühturm erfolgen.

Besonders bewährt zur Herstellung der Träger mit bimodaler Porenradienverteilung hat sich die Verwendung von PVP (Polyvinylpyrrolidon). Wird dieses in einem Herstellschritt zu einem oder mehreren Oxid-Precursoren für Oxide der Elemente Zr, Ti, Al oder Si gegeben, so entstehen nach dem Calcinieren Makroporen im Bereich von 200 bis 5000 nm. Ein weiterer Vorteil der Verwendung von PVP ist die leichtere Verformbarkeit des Trägers. So können aus frisch gefälltem wasserhaltigem ZrO₂•x H₂O, das vorher bei 120°C getrocknet wurde, unter Zusatz von PVP und Ameisensäure auch ohne weitere Oxid-Precursoren mühelos Stränge mit guten mechanischen Eigenschaften hergestellt werden.

Die Calcinierung der Träger für die Dehydrierungskatalysatoren erfolgt zweckmäßigerweise nach dem Aufbringen der Aktivkomponenten und wird bei Temperaturen von 400 bis 1000°C, bevorzugt von 500 bis 700°C, besonders bevorzugt bei 550 bis 650°C und insbesondere bei 560 bis 620°C durchgeführt. Die Calcinierungstemperatur sollte dabei üblicherweise mindestens so hoch sein wie die Reaktionstemperatur der Dehydrierung, bei welcher die erfindungsgemäßen Dehydrierungskatalysatoren eingesetzt werden.

Die Träger der Dehydrierungskatalysatoren weisen nach der Calcinierung im allgemeinen hohe BET-Oberflächen auf. Die BET-Oberflächen sind im allgemeinen größer als 40 m²/g, bevorzugt größer als 50 m²/g, besonders bevorzugt größer als 70 m²/g. Das Porenvolumen der erfindungsgemäßen Dehydrierungskatalysatoren beträgt üblicherweise 0,2 bis 0,6 ml/g, bevorzugt 0,25 bis 0,5 ml/g. Der durch Hg-Porosimetrie bestimmbare mittlere Porendurchmesser der erfindungsgemäßen Dehydrierungskatalysatoren liegt zwischen 3 und 20 nm, bevorzugt zwischen 4 und 15 nm.

Die eingesetzten Dehydrierungskatalysatoren weisen bevorzugt eine bimodale Porenradienverteilung auf. Die Porenradien liegen dabei im Bereich bis 20 nm und zwischen 40 und 5000 nm. Bezogen auf das gesamte Porenvolumen des Dehydrierungskatalysators besitzen diese Poren in Summe mindestens einen Anteil von 70%. Der Anteil an Poren mit Porenradien kleiner als 20 nm beträgt dabei im allgemeinen zwischen 20 und 60%, der Anteil an Poren mit Porenradien zwischen 40 und 5000 nm beträgt im allgemeinen ebenfalls 20 bis 60%.

Die Aufbringung der dehydrieraktiven Komponente, die üblicherweise ein Metall der VIII. Nebengruppe ist, erfolgt in der Regel durch Tränkung mit einem geeigneten Metallsalzprecursor. Statt durch Tränkung kann die dehydrieraktive Komponente aber auch durch andere Verfahren wie beispielsweise Aufsprühen des Metallsalzprecursors erfolgen. Geeignete Metallsalzprecursoren sind z.B. die Nitrate, Acetate und Chloride der entsprechenden Metalle, möglich sind auch komplexe Anionen der verwendeten Metalle. Bevorzugt wird Platin als H₂PtCl₆ oder Pt(NO₃)₂ eingesetzt. Als Lösungsmittel für die Metallsalzprecursoren eignen sich Wasser genauso wie organische Lösungsmittel. Besonders geeignet sind Wasser und niedere Alkohole wie Methanol und Ethanol.

Geeignete Precursoren bei der Verwendung von Edelmetallen als dehydrieraktive Komponente sind auch die entsprechenden Edelmetallsole, die nach einem der bekannten Verfahren, zum Beispiel durch Reduktion eines Metallsalzes in Gegenwart eines Stabilisators wie PVP mit einem Reduktionsmittel hergestellt werden können. Die Herstelltechnik wird in der deutschen Patentanmeldung DE 195 00 366 ausführlich behandelt.

Der Gehalt der Dehydrierungskatalysatoren an einem Edelmetall als dehydrieraktiver Komponente beträgt 0 bis 5 Gew.-%, bevorzugt 0,05 bis 1 Gew.-%, besonders bevorzugt 0,05 bis 0,5 Gew.-%.

Die weiteren Komponenten der Aktivmasse können entweder während der Herstellung des Trägers, zum Beispiel durch gemeinsame Fällung, oder nachträglich, zum Beispiel durch Tränken des Trägers mit geeigneten Precursor-Verbindungen, aufgebracht werden. Als Precursor-Verbindungen verwendet man in der Regel Verbindungen, die sich durch Calcinieren in die entsprechenden Oxide umwandeln lassen. Geeignet sind zum Beispiel

Hydroxide, Carbonate, Oxalate, Acetate, Chloride oder gemischte Hydroxycarbonate der entsprechenden Metalle.

In vorteilhaften Ausführungsformen enthält die Aktivmasse folgende weitere Komponenten:
- mindestens ein Element aus der I. oder II. Hauptgruppe, bevorzugt Caesium und/oder Kalium mit einem Gehalt zwischen 0 und 20 Gew.-%, bevorzugt zwischen 0,1 und 15 Gew.-%, besonders bevorzugt zwischen und 0,2 und 10 Gew.-%;
- mindestens ein Element der III. Nebengruppe einschließlich der Lanthaniden und Actiniden, bevorzugt Lanthan und/oder Cer mit einem Gehalt zwischen 0 und 20 Gew.-%, bevorzugt zwischen zwischen 0,1 und 15 Gew.-%, besonders bevorzugt zwischen und 0,2 und 10 Gew.-%;
- mindestens ein Element aus der III. und IV. Hauptgruppe, bevorzugt Zinn mit einem Gehalt zwischen 0 und 10 Gew.-%.
Der Dehydrierungskatalysator ist bevorzugt halogenfrei.

Der Dehydrierungskatalysator kann im Reaktor fest angeordnet oder z.B. in Form eines Wirbelbettes verwendet werden und eine entsprechende Gestalt haben. Geeignet sind z.B. Formen wie Splitt, Tabletten, Monolithe, Kugeln, oder Extrudate (Stränge, Wagenräder, Sterne, Ringe).

Als dehydrierbare Alkane werden geradkettige oder verzweigte Alkane mit 2 bis 30 C-Atomen eingesetzt. Das Verfahren ist dabei besonders geeignet für die Dehydrierung von gerad- oder verzweigtkettigen Alkanen mit einer Kettenlänge von 2 bis 15 Kohlenstoffatomen, bevorzugt mit 2 bis 5 Kohlenstoffatomen. Beispiele sind Ethan, Propan, n-Butan, iso-Butan, n-Pentan, iso-Pentan, n-Hexan, n-Heptan, n-Octan, n-Nonan, n-Decan, n-Undecan, n-Dodecan, n-Tridecan, n-Tetradecan, n-Pentadecan und deren Gemische. Besonders bevorzugtes Alkan ist Propan.

Da die Dehydrierreaktion unter Volumenzunahme verläuft, kann der Umsatz durch Erniedrigung des Partialdrucks der Reaktanten gesteigert werden. Dies lässt sich in einfacher Weise z.B. durch Dehydrierung bei vermindertem Druck und/oder durch Zumischen eines Inertgases erreichen. Geeignete Inertgase stellen z.B. Stickstoff, Wasserdampf, Kohlendioxid und Edelgase wie He, Ne oder Ar dar. Bevorzugt sind unter den Reaktionsbedingungen inerte (d.h. zu weniger als 5 mol-%, bevorzugt zu weniger als 3 mol-% und noch besser zu weniger als 1 mol-% sich chemisch verändernde) Verdünnungsmittel. Eine Verdünnung mit Wasserdampf bedingt als weiteren Vorteil in der Regel ein vermindertes Verkoken des erfindungsgemäß eingesetzten Dehydrierungskatalysators und damit eine erhöhte Standzeit, da der Wasserdampf mit gebildetem Koks nach dem Prinzip der Kohlevergasung abreagiert. Das Verhältnis von Wasserdampf zu dem zu dehydrierenden Kohlenwasserstoff liegt dabei im Bereich zwischen 0 und 10 mol/mol, bevorzugt zwischen 0,1 und 5 mol/mol.

Das in dem zweiten Teilverfahren des erfindungsgemäßen Verfahren eingesetzte Alkan muss kein Reinstoff sein. Vielmehr kann das Alkan Gase wie Methan, Ethylen, Propen, Butene, Propin, Acetylen oder H₂S enthalten. Insbesondere kann die erfindungsgemäße Dehydrierung auch mit großtechnisch erzeugten und in großen Mengen zur Verfügung stehenden Alkan-Gemischen wie LPG (liquified petroleum gas) durchgeführt werden.

Die Aufarbeitung des Reaktoraustrags erfolgt in an sich bekannter Weise, beispielsweise durch Abtrennung des im Produktgemisch enthaltenen molekularen Wasserstoffs, Abtrennung der von Alkanen und Alkenen verschiedenen Bestandteile, bevorzugt durch selektive Absorption des Alken/Alkan-Gemischs in einem organischen Lösungsmittel und Auftrennung des Alken/Alkan-Gemischs in einem C₃-Splitter und Rückführung des Alkans in die Dehydrierung.

Das erfindungsgemäße Verfahren kann für Propan als zu dehydrierendem Alkan als erste Stufe eines Verfahrens zur Herstellung von Acrolein und/oder Acrylsäure aus Propan durchgeführt werden. Dabei wird Propan einer autothermen Dehydrierung unterworfen, wobei ein Gasgemisch enthaltend Propan und Propen erhalten wird, aus dem Produktgasgemisch der Dehydrierung Propan und Propen abgetrennt und dieses Gemisch einem Oxidationsreaktor zugeführt, in dem Propen einer selektiven heterogen-katalysierten Gasphasenoxidation mit Sauerstoff zu Acrolein und/oder Acrylsäure unterworfen wird. Aus dem Produktgasgemisch der Oxidation wird Acrolein/Acrylsäure abgetrennt und das verbleibende Gasgemisch, das u. a. nicht umgesetztes Propan enthält, in die Alkan-Dehydrierung zurückgeführt. Entsprechend kann das erfindungsgemäße Verfahren für Ethan als zu dehydrierendem Alkan als erste Stufe eines Verfahrens zur Herstellung von Ethylbenzol aus Ethan und Benzol, für Propan als zu dehydrierendem Alkan als erste Stufe eines Verfahrens zur Herstellung von Cumol aus Propan und Benzol und für höhere Alkane als erste Stufe eines Verfahrens zur Herstellung linearer Alkylbenzole aus den höheren Alkanen und Benzol durchgeführt werden. Das nach Produktabtrennung verbleibende Gasgemisch, das u. a. nicht umgesetztes Alkan enthält, kann jeweils in die Alkan-Dehydrierung zurückgeführt werden.

## Patentansprüche

1. Verfahren zur Dehydrierug von C₂-C₃₀-Alkanen, bei dem
(i) in einem ersten Teilverfahren Ethylbenzol zu Styrol dehydriert wird, wobei ein wasserstoffhaltiger Abgasstrom gewonnen wird, und
(ii) in einem zweiten Teilverfahren ein oder mehrere C₂-C₃₀-Alkane in einer oder mehreren Reaktionszonen heterogen katalysiert zu den entsprechenden Olefinen dehydriert werden, wobei dem Reaktionsgasgemisch der Dehydrierung in mindestens einer Reaktionszone ein Wasserstoff enthaltender Gasstrom zugemischt wird und dem Reaktionsgasgemisch der Alkan-Dehydrierung in mindestens einer Reaktionszone zusätzlich Sauerstoff zugemischt und **dadurch** der in dem Reaktionsgasgemisch enthaltene Wasserstoff verbrannt wird, wodurch zumindest ein Teil der benötigten Dehydrierwärme in der mindestens einen Reaktionszone direkt in dem Reaktionsgasgemisch erzeugt wird,
wobei zumindest ein Teil des in der Ethylbenzol-Dehydrierung gewonnenen Wasserstoff enthaltenden Abgasstroms dem Reaktionsgasgemisch der Alkan-Dehydrierung zugemischt wird.

2. Verfahren nach Anspruch 1, **dadurch gekennzeichnet, dass** der Abgasstrom der Ethylbenzol-Dehydrierung, der dem Reaktionsgasgemisch der Alkan-Dehydrierung zugemischt wird, 0,1 bis 4 Vol.-% Kohlenwasserstoffe und 1,5 bis 8 Vol.-% Kohlenstoffoxide enthält.

3. Verfahren nach einem der Anspruch 1 oder 2, **dadurch gekennzeichnet, dass** der Abgasstrom der Ethylbenzol-Dehydrierung, der dem Reaktionsgasgemisch der Alkandehydrierung zugemischt wird, 90 bis 97 Vol.-% Wasserstoff 0,05 bis 0,4 Vol.-% Kohlenmonoxid, 3 bis 7 Vol.-% Kohlendioxid, 0,1 bis 2 Vol.- % Methan, 0,02 bis 0,2 Vol.-% Ethan, 0,1 bis 1,5 Vol.-% Ethylen und insgesamt 0 bis 2 Vol.-% Wasserdampf, Propan, Benzol und/oder Stickstoff enthält, wobei die Summe aus den genannten Komponenten 100 Vol.-% ergibt.

4. Verfahren nach einem der Ansprüche 1 bis 3, **dadurch gekennzeichnet**, das der Alkan-Dehydrierungskatalysator mindestens ein Metalloxid, ausgewählt aus der Gruppe bestehend aus Zirkondioxid, Aluminiumoxid, Siliciumdioxid, Titandioxid, Magnesiumoxid, Lanthanoxid und Ceroxid, mindestens ein Element der VIII. Nebengruppe, mindestens ein Element der L oder II. Hauptguppe, mindestens ein Element der III. oder IV. Hauptgruppe und mindestens ein Element der III. Nebengruppe, einschließlich der Lanthaniden und Actiniden, enthält.

5. Verfahren nach Anspruch 4, **dadurch gekennzeichnet, dass** der Alkan-Dehydrierungskatalysator Zirkondioxid und/oder Siliziumdioxid, Platin und/oder Palladium, Caesium und/oder Kalium, Lanthan und/oder Cer und Zinn enthält.

6. Verfahren nach einem der Ansprüche 1 bis 5, **dadurch gekennzeichnet, dass** wenigstens eine Reaktionszone der Alkan-Dehydrierung einen Katalysator enthält, der selektiv die Verbrennung von Wasserstoff mit Sauerstoff in Gegenwart von Kohlenwasserstoffen katalysiert.

7. Verfahren, nach einem der Ansprüche 1 bis 6, **dadurch gekennzeichnet, dass** dem Reaktionsgasgemisch der Alkan-Dehydrierung Wasserdampf zugemischt wird.

8. Verfahren nach einem der Ansprüche 1 bis 7, **dadurch gekennzeichnet, dass** die Alkan-Dehydnerung in einem Hordenreaktor mit 2 bis 8 Katalysatorbetten durchgeführt wird.

## Claims

1. A process for the dehydrogenation of C₂-C₃₀-alkanes, in which
(i) ethylbenzene is dehydrogenated to styrene in a first part process to give a hydrogen-containing offgas stream, and
(ii) one or more C₂-C₃₀-alkanes are dehydrogenated in the presence of a heterogeneous catalyst in one or more reaction zones in a second part process to give the corresponding olefins, with a hydrogen-comprising gas stream being mixed into the reaction gas mixture of the dehydrogenation in at least one reaction zone and oxygen being additionally mixed into the reaction gas mixture of the alkane dehydrogenation in at least one reaction zone and the hydrogen comprised in the reaction gas mixture thereby being burnt, as a result of which at least part of the necessary heat of dehydrogenation is generated directly in the reaction gas mixture in the reaction zone or zones,
wherein at least part of the hydrogen-comprising offgas stream obtained in the dehydrogenation of ethylbenzene is mixed into the reaction gas mixture of the alkane dehydrogenation.

2. The process according to claim 1, wherein the offgas stream from the ethylbenzene dehydrogenation which is mixed into the reaction gas mixture of the alkane dehydrogenation comprises from 0.1 to 4% by volume of hydrocarbons and from 1.5 to 8% by volume of carbon oxides.

3. The process according to either of claims 1 and 2, wherein the offgas stream from the ethylbenzene dehydrogenation which is mixed into the reaction gas mixture of the alkane dehydrogenation comprises from 90 to 97% by volume of hydrogen, from 0.05 to 0.4% by volume of carbon monoxide, from 3 to 7% by volume of carbon dioxide, from 0.1 to 2% by volume of methane, from 0.02 to 0.2% by volume of ethane, from 0.1 to 1.5% by volume of ethylene and a total of from 0 to 2% by volume of water vapor, propane, benzene and/or nitrogen, where the sum of the components specified is 100% by volume.

4. The process according to any of claims 1 to 3, wherein the alkane dehydrogenation catalyst comprises at least one metal oxide selected from the group consisting of zirconium dioxide, aluminum oxide, silicon dioxide, titanium dioxide, magnesium oxide, lanthanum oxide and cerium oxide, at least one element of transition group VIII, at least one element of main group I or II, at least one element of main group III or IV and at least one element of transition group III including the lanthanides and actinides.

5. The process according to claim 4, wherein the alkane dehydrogenation catalyst comprises zirconium dioxide and/or silicon dioxide, platinum and/or palladium, cesium and/or potassium, lanthanum and/or cerium and tin.

6. The process according to any of claims 1 to 5, wherein at least one reaction zone of the alkane dehydrogenation comprises a catalyst which selectively catalyzes the combustion of hydrogen with oxygen in the presence of hydrocarbons.

7. The process according to any of claims 1 to 6, wherein steam is mixed into the reaction gas mixture of the alkane dehydrogenation.

8. The process according to any of claims 1 to 7, wherein the alkane dehydrogenation is carried out in a tray reactor having from 2 to 8 catalyst beds.

## Revendications

1. Procédé de déshydrogénation d'alcanes en C₂-C₃₀, selon lequel
(i) lors d'un premier procédé partiel, de l'éthylbenzène est déshydrogéné en styrène, un courant de gaz d'échappement contenant de l'hydrogène étant obtenu, et
(ii) lors d'un second procédé partiel, un ou plusieurs alcanes en C₂-C₃₀ sont déshydrogénés dans une ou plusieurs zones de réaction sous catalyse hétérogène pour former les oléfines correspondantes, un courant gazeux contenant de l'hydrogène étant mélangé avec le mélange gazeux réactionnel de la déshydrogénation dans au moins une zone de réaction et de l'oxygène supplémentaire étant mélangé avec le mélange gazeux réactionnel de la déshydrogénation des alcanes dans au moins une zone de réaction et l'hydrogène contenu dans le mélange gazeux réactionnel étant ainsi brûlé, au moins une partie de la chaleur de déshydrogénation nécessaire étant générée directement dans le mélange gazeux réactionnel dans la ou les zones de réaction,
dans lequel au moins une partie du courant de gaz d'échappement contenant de l'hydrogène obtenu lors de la déshydrogénation de l'éthylbenzène est mélangée avec le mélange gazeux réactionnel de la déshydrogénation des alcanes.

2. Procédé selon la revendication 1, **caractérisé en ce que** le courant de gaz d'échappement de la déshydrogénation de l'éthylbenzène qui est mélangé avec le mélange gazeux réactionnel de la déshydrogénation des alcanes contient 0,1 à 4% en volume d'hydrocarbures et 1,5 à 8 % en volume d'oxydes de carbone.

3. Procédé selon l'une quelconque des revendications 1 et 2, **caractérisé en ce que** le courant de gaz d'échappement de la déshydrogénation de l'éthylbenzène qui est mélangé avec le mélange gazeux réactionnel de la déshydrogénation des alcanes contient 90 à 97 % en volume d'hydrogène, 0,05 à 0,4 % en volume de monoxyde de carbone, 3 à 7 % en volume de dioxyde de carbone, 0,1 à 2 % en volume de méthane, 0,02 à 0,2 % en volume d'éthane, 0,1 à 1,5 % en volume d'éthylène et au total 0 à 2 % en volume de vapeur d'eau, de propane, de benzène et/ou d'azote, la somme des composants cités atteignant 100 % en volume.

4. Procédé selon l'une quelconque des revendications 1 à 3, **caractérisé en ce que** le catalyseur de la déshydrogénation des alcanes contient au moins un oxyde métallique, choisi dans le groupe constitué du dioxyde de zirconium, de l'oxyde d'aluminium, du dioxyde de silicium, du dioxyde de titane, de l'oxyde de magnésium, de l'oxyde de lanthane et de l'oxyde de cérium, au moins un élément du sous-groupe VIII, au moins un élément du groupe principal I ou II, au moins un élément du groupe principal III ou IV et au moins un élément du sous-groupe III, y compris les lanthanides et les actinides.

5. Procédé selon la revendication 4, **caractérisé en ce que** le catalyseur de la déshydrogénation des alcanes contient du dioxyde de zirconium et/ou du dioxyde de silicium, du platine et/ou du palladium, du césium et/ou du potassium, du lanthane et/ou du cérium et de l'étain.

6. Procédé selon l'une quelconque des revendications 1 à 5, **caractérisé en ce qu'**au moins une zone de réaction de la déshydrogénation des alcanes contient un catalyseur qui catalyse sélectivement la combustion de l'hydrogène avec l'oxygène en présence d'hydrocarbures.

7. Procédé selon l'une quelconque des revendications 1 à 6, **caractérisé en ce que** de la vapeur d'eau est mélangée avec le mélange gazeux réactionnel de la déshydrogénation des alcanes.

8. Procédé selon l'une quelconque des revendications 1 à 7, **caractérisé en ce que** la déshydrogénation des alcanes est réalisée dans un réacteur à étages qui comprend 2 à 8 lits catalytiques.
